# EUROPÄISCHE PATENTSCHRIFT

(11) **EP 2 839 747 B1**
(45) Veröffentlichungstag und Bekanntmachung des Hinweises auf die Patenterteilung: **05.06.2019**
(21) Anmeldenummer: 13180794.3
(22) Anmeldetag: 18.08.2013
(51) Int. Cl.: A23C 9/12

(54) **Starterkulturen für die Herstellung von fermentierten Milchprodukten**
Starter cultures for the production of fermentated milk products
Cultures bactériennes pour la production des produits de fermentation du lait

(43) Veröffentlichungstag der Anmeldung: 25.02.2015
(73) Patentinhaber: DMK Deutsches Milchkontor GmbH, 27404 Zeven (DE)
(72) Erfinder: Schomacker, Marina, 27419 Sittensen (DE); Hahn, Michael, 27404 Heerlingen (DE)
(74) Vertreter: Fabry, Bernd

(56) Entgegenhaltungen:
- EP-A1- 1 593 309
- EP-A1- 1 752 046
- DE-T2- 60 129 217
- DE-T2- 69 219 130
- US-A1- 2005 208 630
- DATABASE WPI Week 201327 Thomson Scientific, London, GB; AN 2013-D48660 XP002715706, -& KR 2013 0021346 A (YOO S H) 5. März 2013 (2013-03-05)

## Beschreibung

### Gebiet der Erfindung

Die Erfindung befindet sich auf dem Gebiet der Milchprodukte und betrifft Starterkulturen zur Herstellung von fermentierten Produkten, speziell geschmacksverbessertem Quark, ein Verfahren zur Herstellung von Quarkgrundmassen unter Einsatz der Starterkulturen sowie die Verwendung der Mikroorganismen zur Herstellung von Quarkgrundmassen.

### Stand der Technik

Zur Herstellung von Quark wird in der Regel entrahmte Milch einer Temperaturbehandlung unterworfen und die enthaltenen Proteine denaturiert.

Durch die anschließende Zugabe von Milchsäurebakterien und Lab erfolgt die so genannte Dicklegung (Phasenumkehrung) der Milch. Das Casein gerinnt und bildet die fachlich bezeichnete Gallerte aus. Nach der Reifung (8 bis 20 h) wird die Gallerte aufgerührt. Hierdurch wurde die Molkenabtrennung ausgelöst und im Separator werden dann die beiden Phasen getrennt. Die flüssige Sauermolke wird anderweitig verarbeitet und die Quarkgrundmasse durch Zugabe von Sahne auf den gewünschten Fett- und Proteingehalt eingestellt.

Das Dokument DE 692 19 130 T2 offenbart offenbart ein Herstellungsverfahren für ein Hartkäse- oder halbfestes Schnittkäseprodukt aus Milch unter Verwendung einer Kombination einer mesophilen Starterkultur - eine sogenannte BOS-Kultur - mit Joghurt-Bakterien. Die Joghurt-Bakterien umfassen dabei *Streptococcus thermophilus* und *Lactobacillus bulgaricus* in einem Gewichtsverhältnis der mesophilen Kultur zu Joghurt-Bakterien von 0,30 bis 0,02.

Das Dokument KR 2013 0021346 A offenbar ein Verfahren zur Herstellung eines fermentierten Joghurts mit Hilfe eines *acidophilus-bifidus-thermophilus* (ABT) Starters und eines Kefirstammes.

Das Dokument EP 1 593 309 A1 offenbart die Kombination einer Joghurtkultur mit einer Quarkkultur für die Herstellung von Milchprodukten aus einem sogenannten *"starter medium".* Vorzugsweise ist das *"starter medium"* Milch.

Das Dokument US 2005/208630 A1 offenbart einen neuen Stamm von *Streptococcus thermophilus, Streptococcus thermophilus* ST 111, und seine Anwendung bei der Herstellung von stabilen hochmolekularen Heteropolysacchariden.

Das Dokument DE 601 29 217 T2 offenbart ein Verfahren zur Herstellung von Basisquark ausgehend von Buttermilch. Süße Buttermilch wird zuerst 2 bis 4 Minuten bei 82-85 °C erhitzt. Die dabei erhaltene pasteurisierte süße Buttermilch wird in einem Behälter auf 22 bis 24 °C abgekühlt. Dann wird die süße Buttermilch mittels der 0,6 bis 1% Säuerungskultur Zugabe angesäuert. Danach wird das "Lab" zugegeben, um auf diese Weise die pasteurisierte Buttermilch für 14 bis 16 Stunden reifen zu lassen, bis ein pH von 4,0 bis 4,7 erreicht wird. Die Mischung wird gerührt und danach langsam unter langsamem Rühren auf eine Temperatur von 40 bis 50 °C erhitzt. Anschließend wird die Mischung in einem Separator in Quark und Molke separiert.

Das Dokument EP 1 752 046 A1 offenbart ein Verfahren, dessen erfinderischer Gedanke darin besteht, dass die fermentierte Prozessmilch als zu filtrierendes Rohprodukt in an sich bekannter Weise in einem Mikrofiltrationsverfahren weiterverarbeitet wird. Das Mikrofiltrationsverfahren arbeitet dabei als Querstrom- oder Cross-flow-Filtration und seine trennspezifischen Bedingungen an den Membranen sind derart festgelegt, dass an den Membranen eine spezielle Retentatdeckschicht entsteht. Bei den trennspezifischen Bedingungen handelt es sich im Wesentlichen um den Porendurchmesser der Membranen, den Transmembrandruck über die eigentliche Membran, die Überströmrate und die Arbeitstemperatur.

Diese Verfahren des Stands der Technik haben jedoch den Nachteil, dass die sensorische Beurteilung des Quarks von vielen Konsumenten als nicht zufriedenstellend beurteilt wird. Entweder ist das Produkt geschmacklich einwandfrei, weist aber eine rauhe Struktur auf, oder es ist vom haptischen Eindruck her zwar cremig, hinterlässt aber einen schleimigen Gesamteindruck.

Die komplexe Aufgabe der vorliegenden Erfindung hat somit darin bestanden, eine Quarkmasse mit geschmacklich verbesserten Eigenschaften zur Verfügung zu stellen, die beim Verzehr gerade einen cremigen Eindruck hinterlässt, ohne dass das Produkt schleimig schmeckt.

### Beschreibung der Erfindung

Ein erster Gegenstand der Erfindung betrifft Starterkulturen für die Herstellung von fermentierten Milchprodukten, die sich dadurch auszeichnen, dass sie
(a) 25 bis 75 Gew.-% einer ersten Mischung von fünf Mikroorganismen umfassend (a1) *Streptococcus thermophilus,* (a2) *Leuconostoc species,* (a3) *Lactococcus lactis subsp. lactis biovar diacetylactis,* (a4) *Lactococcus lactis subsp. lactis* und (a5) *Lactococcus lactis subsp. cremoris,* und
(b) 75 bis 25 Gew.-% einer zweiten Mischung von drei Mikroorganismen umfassend (b1) *Streptococcus thermophilus,* (b2) *Lactococcus lactis subsp. lactis* und (b3) *Lactococcus lactis subsp. cremoris,* sowie
(c) 0 bis 10 Gew.-% einer dritten Gruppe von Mikroorganismen umfassend (c1) *Bifido bakterium lactis B12,* (c2) *Lactobazillus acidophilus,* (c3) *Leuconostoc cremoris* oder deren Mischungen
mit der Maßgabe enthalten, dass sich die Mengenangaben zu 100 Gew.-% ergänzen,
wobei die fünf Mikroorganismen der Mischung (a) jeweils in Mengen von 20 ± 5 Gew.-% und die drei Mikroorganismen der Mischung (b) jeweils in Mengen von 33 ± 5 Gew.-% enthalten sind.

Bei der Gruppe (c) handelt es sich um Mikroorganismen, die üblicherweise als Starterkulturen zur Quarkherstellung Verwendung finden und im Stand der Technik umfangreich beschrieben sind. Mit diesen Kulturen lassen sich indes lediglich die eingangs beschriebenen Produkte mit den unbefriedigenden geschmacklichen und sensorischen Eigenschaften herstellen, so dass die resultierenden Grundmassen umfangreich nachbearbeitet werden müssen, ehe sie als verzehrsfähige Produkte in den Markt gelangen.

Die Anmelderin hat nun in umfangreichen Versuchsreihen mit den unterschiedlichsten Milchsäurebakterien eine kleine Gruppe von Stämmen identifiziert, die über besonders vorteilhafte Eigenschaften verfügen.

So wurde nun überraschenderweise gefunden, dass die Kombination der Mikroorganismen der Gruppen (a) und (b) zu einer deutlichen Verbesserung der sensorischen und geschmacklichen Eigenschaften der Quarkgrundmassen führt, so dass unmittelbar Quarkgrundmassen erhalten werden, die verzehrsfähig sind und keiner Nachbehandlung, wie Süßen oder Stretching etc. mehr bedürfen. Diese Starterkulturmischungen tolerieren dazu in gewissem Umfang auch die konventionellen Starterkulturen der Gruppe (c).

### Starterkulturen

Wie erläutert betrifft die Erfindung Starterkulturen, die mindestens zwei unterschiedliche Mischungen von Mikroorganismen umfassen, wobei die Gruppen (a) und (b) von ihren Inhaltsstoffe überlappen. Der Grund hierfür ist, dass Starterkulturen in der Regel nicht als reine Stämme, sondern jeweils selbst als Mischungen im Handel erhältlich sind. Beim Einsatz der Startermischung (a) alleine ergäbe sich im Endprodukt ein quarkuntypisches Geschmacksprofil mit einer ausgeprägten buttrigen Note. Erst die Kombination der Mischungen (a) und (b) führt zu einem Produkt, das geschmacklich einwandfrei ist.

Vorzugsweise enthalten die Starterkulturen
(i) 40 bis 60 Gew.-% der Mischung (a),
(ii) 60 bis 40 Gew.-% der Mischung (b) und
(iii) 0 bis 10 Gew.-% und insbesondere 5 bis 10 Gew.-% der Mischung (c)
mit der Maßgabe, dass sich die Mengenangaben zu 100 Gew.-% ergänzen.

Besonders bevorzugt sind Starterkulturen, die
(i) 45 bis 55 Gew.-% der Mischung (a) und
(ii) 55 bis 45 Gew.-% der Mischung (b)
mit der Maßgabe enthalten, dass sich die Mengenangaben zu 100 Gew.-% ergänzen.

Statt die beiden im Handel erhältlichen Zubereitungen (a) und (b) gemeinsam einzusetzen, ist es natürlich grundsätzlich auch möglich, die fünf Mikroorganismen einzeln zu verwenden und dann so abzumischen, dass eine Starterkulturmischung erhalten wird, mit der die geschmacklich verbesserten Quarkprodukte erhalten werden. Solche Starterkulturen enthalten dann vorzugsweise
(i) 20 bis 30 Gew.-% *Streptococcus thermophilus,*
(ii) 5 bis 15 Gew.-% *Leuconostoc species,*
(iii) 5 bis 10 Gew.-% *Lactococcus lactis subsp. lactis biovar diacetylactis,*
(iv) 20 bis 30 Gew.-% *Lactococcus lactis subsp. lactis,*
(v) 20 bis 30 Gew.-% *Lactococcus lactis subsp. cremoris,* und
(vi) 0 bis 15 Gew.-% Bifido bakterium lactis B12,
mit der Maßgabe, dass sich die Mengenangaben zu 100 Gew.-% ergänzen.

Besonders bevorzugt sind Starterkulturen enthaltend
(i) 25 Gew.-% *Streptococcus thermophilus,*
(ii) 12 Gew.-% *Leuconostoc species,*
(iii) 13 Gew.-% *Lactococcus lactis subsp. lactis biovar diacetylactis,*
(iv) 25 Gew.-% *Lactococcus lactis subsp. lactis,*
(v) 25 Gew.-% *Lactococcus lactis subsp. cremoris*

Alle genannten Mikroorganismen sind im Handel frei verfügbar.

### Quarkgrundmassen und deren Herstellung

Des Weiteren ist die Erfindung auf ein Verfahren zur Herstellung einer Quarkgrundmasse mit verbesserten Geschmackseigenschaften gerichtet, bei dem man
(a) Rohmilch einer Temperaturbehandlung unterwirft und den Rahm abtrennt, so dass eine ungesäuerte Quarkgrundmasse entsteht,
(b) die so erhaltene Mischung einer Temperaturbehandlung unterwirft, bis Denaturierung eintritt,
(c) das denaturierte Produkt mit den erfindungsgemäßen Starterkulturen und Lab versetzt und gegebenenfalls
(d) die nach Abschluss der Fermentation erhaltene Quarkgrundmasse auf einen definierten Trockenmassen- und Proteingehalt einstellt

Vorzugsweise weist die Quarkgrundmasse bei 20 °C eine Viskosität nach Brookfield (RVT, Spindel 1, 10 Upm) im Bereich von etwa 1.000 bis etwa 8.000 mPas, vorzugsweise etwa 2.000 bis etwa 6.00 mPas und insbesondere etwa 3.000 bis etwa 5.000 mPas auf.

Zur Herstellung der ungesäuerten Quarkgrundmassse erfolgt zunächst eine Abtrennung von Feststoffen sowie die Entrahmung des Fettanteils von etwa 4 Gew.-% aus der Rohmilch. Dies findet üblicherweise in einem speziellen Bauteil, vorzugsweise einem Separator statt. Derartige Bauteile sind aus dem Stand der Technik hinreichend bekannt. In der Milchindustrie sehr verbreitet sind Separatoren der Firma GEA Westfalia Separator GmbH, mit denen die beiden Schritte einzeln oder gemeinsam durchgeführt werden können.¹ Entsprechende Bauteile sind beispielsweise auch in der DE 10036085 C1 (Westfalia) beschrieben und dem Fachmann bestens bekannt, so dass es zur Durchführung dieser Verfahrensschritte keiner Erläuterungen bedarf, da sie den allgemeinen Fachwissen zuzurechnen sind.
¹ (http://www.westfalia-separator.com/de/anwendungen/molkereitechnik/milch-molke.html).

Die Wärmebehandlung der Rohmilch erfolgt vorzugsweise in Wärmeaustauschern, wobei sich speziell Plattenwärmeaustauscher als besonders geeignet erwiesen haben. An den Wärmeaustauschern liegt ein Temperaturgradient an, der jedoch so gewählt ist, dass die Rohmilch für eine Verweilzeit von mindestens 20 und höchstens 60 Sekunden, vorzugsweise etwa 30 Sekunden auf eine Temperatur von etwa 70 bis 80 °C und insbesondere etwa 72 bis 74 °C erhitzt wird.

Im folgenden Schritt wird die so erhaltene ungesäuerte Quarkgrundmasse einer thermischen Behandlung unterworfen. Die nun erfolgende Denaturierung kann in an sich bekannter Weise erfolgen, nämlich über einen Zeitraum von 5 bis 10 min und vorzugsweise 6 min und Temperaturen von 85 bis 90 °C und insbesondere 88 °C.

Auch die Fermentation des denaturierten Vorproduktes kann nach den bekannten Verfahren des Stands der Technik erfolgen. Dazu werden die erfindungsgemäßen Starterkulturen und Lab zugesetzt.

Die Temperatur, bei der die Fermentation erfolgt, richtet sich nach dem Temperaturbereich, der für die jeweilig verwendeten Mikroorganismen optimal ist; typisch liegt die Temperatur im Bereich von 18 bis 35 °C und vorzugsweise bei 30 °C.

Die nach der Fermentation erhaltene Quarkgrundmasse wird anschließend beispielsweise durch Zugabe von Sahne auf den gewünschten Gehalt an Trockenmasse und Proteinen eingestellt. Vorzugsweise liegt der Trockenmassengehalt bei 15 bis 20 Gew.-% und insbesondere bei 18 Gew.-%. Der Proteingehalt kann 10 bis 15 Gew.-% und vorzugsweise 12 Gew.-% betragen.

### Beispiele

### Beispiele 1 bis 3, Vergleichsbeispiele V1 bis V5

4 kg Magermilch wurden 6 min bei 88 °C behandelt und die enthaltenen Proteine denaturiert. Die Masse wurde mit unterschiedlichen Starterkulturen und Lab versetzt, bei 30 °C etwa 18 h gereift und anschließend aufgerührt. Anschließend wurde das Fermentationsprodukt in eine Zentrifuge gegeben und ca. 3,2 kg Sauermolke als flüssiger Bestandteil abgetrennt. Die zurückgebliebene Quarkmasse (ca. 800 g) wurde durch Zugabe von Sahne auf eine Trockenmasse von 18 Gew.-% und einen Proteingehalt von 12 Gew.-% eingestellt. Anschließend wurden die Produkte von einem Panel bestehend aus 5 erfahrenen Testern geschmacklich und sensorisch auf einer Skala von 1 (= trifft nicht zu) bis 6 (= trifft voll zu) beurteilt. Die Ergebnisse sind in **Tabelle 1** zusammengefasst. Die Beispiele 1 bis 3 sind erfindungsgemäß, die Beispiele V1 bis V5 dienen zum Vergleich. Angegeben sind die Mittelwerte der Beurteilungen.

**Tabelle 1**

| Geschmackliche und sensorische Beurteilung der Quarkgrundmassen | | | | | |
|---|---|---|---|---|---|
| **Bsp.** | **Starterkultur** | **Geschmack** | **Sensorik** | | |
| | | **Bitter** | **Cremig** | **Glatt** | **Schleimig** |
| V1 | *Bifido bakterium lactis B*12 | 5,5 | 3,0 | 2,0 | 5,5 |
| V2 | *Lactobazillus acidophilus* | 5,0 | 3,5 | 2,0 | 5,5 |
| V3 | *Bifido bakterium lactis B12* + *Lactobazillus acidophilus (1:1)* | 5,0 | 3,0 | 2,0 | 5,5 |
| V4 | Mischung (a) | 3,0 | 2,5 | 2,0 | 4,0 |
| V5 | Mischung (b) | 4,0 | 2,0 | 2,0 | 4,0 |
| 1 | Mischung (a+b) = 75:25 | 2,0 | 4,0 | 3,5 | 2,0 |
| 2 | Mischung (a+b) = 50:50 | 1,5 | 4,5 | 4,0 | 1,0 |
| 3 | Mischung (a+b) = 25:75 | 2,5 | 4,0 | 3,5 | 1,5 |

Die Versuche und Vergleichsversuche zeigen deutlich, dass die Auswahl der Starterkulturen einen erheblichen Einfluss auf die geschmacklichen und sensorischen Eigenschaften der Quarkgrundmasse hat.

Die Vergleichsbeispiele V1 bis V5 wurden mit klassischen Milchsäurebakterien sowie mit den beiden Mischungen (a) und (b) jeweils alleine durchgeführt. Die Quarkgrundmassen schmeckten überwiegend bitter und wiesen mehrheitlich eine schleimige Konsistenz auf; die Massen waren zudem wenig cremig und glatt.

Durch Kombination von der Starterkulturmischungen (a) und (b) werden hingegen unmittelbar Quarkgrundmassen erhalten, die ausgezeichnet cremig und glatt erschienen, keine schleimige Konsistenz mehr besaßen und auch kaum bitteren Geschmack aufwiesen.

## Patentansprüche

1. Starterkulturen für die Herstellung von fermentierten Milchprodukten, **dadurch gekennzeichnet, dass** sie
(a) 25 bis 75 Gew.-% einer ersten Mischung von fünf Mikroorganismen umfassend (a1) *Streptococcus thermophilus,* (a2) *Leuconostoc species,* (a3) *Lactococcus lactis subsp. lactis biovar diacetylactis,* (a4) *Lactococcus lactis subsp. lactis* und (a5) *Lactococcus lactis subsp. cremoris,* und
(b) 75 bis 25 Gew.-% einer zweiten Mischung von drei Mikroorganismen umfassend (b1) *Streptococcus thermophilus,* (b2) *Lactococcus lactis subsp. lactis* und (b3) *Lactococcus lactis subsp. cremoris,* sowie
(c) 0 bis 10 Gew.-% einer dritten Gruppe von Mikroorganismen umfassend (c1) *Bifido bakterium lactis B12,* (c2) *Lactobazillus acidophilus,* (c3) *Leuconostoc cremoris* oder deren Mischungen
mit der Maßgabe enthalten, dass sich die Mengenangaben zu 100 Gew.-% ergänzen,
wobei die fünf Mikroorganismen der Mischung (a) jeweils in Mengen von 20 ± 5 Gew.-% und die drei Mikroorganismen der Mischung (b) jeweils in Mengen von 33 ± 5 Gew.-% enthalten sind.

2. Starterkulturen nach Anspruch 1, **dadurch gekennzeichnet, dass** sie
(i) 40 bis 60 Gew.-% der Mischung (a),
(ii) 60 bis 40 Gew.-% der Mischung (b) und
(iii) 0 bis 10 Gew.-% der Mischung (c)
mit der Maßgabe enthalten, dass sich die Mengenangaben zu 100 Gew.-% ergänzen.

3. Starterkulturen nach Anspruch 1, **dadurch gekennzeichnet, dass** sie
(i) 45 bis 55 Gew.-% der Mischung (a) und
(ii) 55 bis 45 Gew.-% der Mischung (b)
mit der Maßgabe enthalten, dass sich die Mengenangaben zu 100 Gew.-% ergänzen.

4. Starterkulturen nach mindestens einem der Ansprüche 1 bis 3, **dadurch gekennzeichnet, dass** sie
(i) 20 bis 30 Gew.-% *Streptococcus thermophilus,*
(ii) 5 bis 15 Gew.-% *Leuconostoc species,*
(iii) 5 bis 10 Gew.-% *Lactococcus lactis subsp. lactis biovar diacetylactis,*
(iv) 20 bis 30 Gew.-% *Lactococcus lactis subsp. lactis,*
(v) 20 bis 30 Gew.-% *Lactococcus lactis subsp. cremoris,* und
(vi) 0 bis 15 Gew.-% Bifido bakterium lactis B12,
mit der Maßgabe enthalten, dass sich die Mengenangaben zu 100 Gew.-% ergänzen.

5. Starterkulturen nach mindestens einem der Ansprüche 1 bis 3, **dadurch gekennzeichnet, dass** sie
(i) 25 Gew.-% *Streptococcus thermophilus,*
(ii) 12 Gew.-% *Leuconostoc species,*
(iii) 13 Gew.-% *Lactococcus lactis subsp. lactis biovar diacetylactis,*
(iv) 25 Gew.-% *Lactococcus lactis subsp. lactis,*
(v) 25 Gew.-% *Lactococcus lactis subsp. cremoris*
enthalten.

6. Verfahren zur Herstellung einer Quarkgrundmasse mit verbesserten Geschmackseigenschaften, bei dem man
(a) Rohmilch einer Temperaturbehandlung unterwirft und den Rahm abtrennt, so dass eine ungesäuerte Quarkgrundmasse entsteht,
(b) die so erhaltene Mischung einer Temperaturbehandlung unterwirft, bis Denaturierung eintritt,
(c) das denaturierte Produkt mit Starterkulturen nach Anspruch 1 und Lab versetzt und gegebenenfalls
(d) die nach Abschluss der Fermentation erhaltene Quarkgrundmasse auf einen definierten Trockenmassen- und Proteingehalt einstellt.

7. Verfahren nach Anspruch 6, **dadurch gekennzeichnet, dass** man die Quarkgrundmasse über einen Zeitraum von 5 bis 10 min einer Temperaturbehandlung bei 85 bis 90 °C unterwirft und dabei denaturiert.

8. Verfahren nach den Ansprüchen 6 und/oder 7, **dadurch gekennzeichnet, dass** man die so erhaltene denaturierte Masse bei 18 bis 35 °C mit den Starterkulturen und Lab versetzt.

9. Verfahren nach mindestens einem der Ansprüche 6 bis 8, **dadurch gekennzeichnet, dass** man die resultierende Quarkgrundmasse auf eine Trockenmasse von 15 bis 20 Gew.-% und einen Proteingehalt von 10 bis 15 Gew.-% einstellt.

10. Verwendung von Mischungen von Mikroorganismen nach Anspruch 1 als Starterkulturen zur Herstellung von Quark.

## Claims

1. Starter cultures for the production of fermented milk products, **characterized in that** they contain
(i) 25 to 75% by weight of a first mixture of five microorganisms comprising (i-1) *Streptococcus thermophilus,* (i-2) *Leuconostoc species,* (i-3) *Lactococcus lactis subsp. lactis biovar diacetylactis,* (i-4) *Lactococcus lactis subsp. lactis* and (i-5) *Lactococcus lactis subsp. cremoris,* and
(ii) 75 to 25% by weight of a second mixture of three microorganisms comprising (ii-1) *Streptococcus thermophilus,* (ii-2) *Lactococcus lactis subsp. lactis* and (ii-3) *Lactococcus lactis subsp. Cremoris,* as well as
(iii) 0 to 10% by weight of a third group of microorganisms comprising (c1) *Bifido bakterium lactis B12,* (c2) *Lactobazillus acidophilus,* (c3) *Leuconostoc cremoris* or mixtures thereof
with the proviso that the amounts add up to 100% by weight,
and wherein in the mixture (i) the five microorganisms are each present in amounts of 20 ± 5% by weight and in the mixture (ii) the three microorganisms are each present in amounts of 33 ± 5% by weight.

2. Starter cultures according to claim 1 **characterized in that** they contain
(i) 40 to 60% by weight of the mixture (a),
(ii) 60 to 40% by weight of the mixture (b) and
(iii) 0 to 10% by weight of a mixture (c)
with the proviso that the amounts add up to 100% by weight.

3. Starter cultures according to claim 1 **characterized in that** they contain
(i) 45 to 55% by weight of the mixture (a), and
(ii) 55 to 45% by weight of the mixture (b)
with the proviso that the amounts add up to 100% by weight.

4. Starter cultures according to at least one of claims 1 to 3 **characterized in that** they contain
(i) 20 to 30% by weight *Streptococcus thermophilus,*
(ii) 5 to 15% by weight *Leuconostoc species,*
(iii) 5 to 10% by weight *Lactococcus lactis subsp. lactis biovar diacetylactis,*
(iv) 20 to 30% by weight *Lactococcus lactis subsp. lactis,*
(v) 20 to 30% by weiht *Lactococcus lactis subsp. cremoris,* and
(vi) 0 to 15% by weight *Bifido bakterium lactis B12,*
with the proviso that the amounts add up to 100% by weight.

5. Starter cultures according to at least one of claims 1 to 3 **characterized in that** they contain
(i) 25% by weight *Streptococcus thermophilus,*
(ii) 12% by weight *Leuconostoc species,*
(iii) 13% by weight *Lactococcus lactis subsp. lactis biovar diacetylactis,*
(iv) 25% by weight *Lactococcus lactis subsp. lactis,*
(v) 25% by weiht *Lactococcus lactis subsp. cremoris.*

6. A method for the production of a quark base composition with improved taste characteristics, wherein
(a) raw milk is subjected to a temperature treatment and the cream is separated off in such a manner that a non-acidified quark base mix is formed,
(b) the resultant mixture is subjected to a temperature treatment until denaturation occurs,
(c) the denatured product is admixed with starter culture according to claim 1 and rennet and optionally,
(d) the quark base mix obtained after completion of fermentation is adjusted to a defined dry matter content and to a protein content.

7. The method according to claim 6, wherein the quark base composition is subjected over a time period from 5 to 10 min to a temperature treatment at 85 to 90 °C and is denatured thereby.

8. Method according to claims 6 and/or 7, wherein the obtained denatured mass is mixed with the starter culture and rennet at 18 to 35 °C.

9. Method according to at least one of claims 6 to 8, wherein in the resultant quark base composition, the dry matter content is adjusted to 15 to 20% by weight and the protein content is adjusted to 10 to 15% by weight.

10. Use of the mixtures of microorganisms according to claim 1 as starter cultures for producing quark.

## Revendications

1. Cultures de départ pour la fabrication de produits laitiers fermentés, **caractérisées en ce qu'**elles contiennent :
(a) 25 à 75 % en poids d'un premier mélange de cinq microorganismes comprenant (a1) *Streptococcus thermophilus,* (a2) *Leuconostoc species,* (a3) *Lactococcus lactis subsp. lactis biovar diacetylactis,* (a4) *Lactococcus lactis subsp. lactis* et (a5) *Lactococcus lactis subsp. cremoris,* et
(b) 75 à 25 % en poids d'un deuxième mélange de trois microorganismes comprenant (b1) *Streptococcus thermophilus,* (b2) *Lactococcus lactis subsp. lactis* et (b3) *Lactococcus lactis subsp. cremoris,* et
(c) 0 à 10 % en poids d'un troisième groupe de microorganismes comprenant (c1) *Bifido bacterium lactis B12,* (c2) *Lactobacillus acidophilus,* (c3) *Leuconostoc cremoris* ou leurs mélanges,
à condition que la somme des données de quantités soit de 100 % en poids,
les cinq microorganismes du mélange (a) étant chacun contenus en quantités de 20 ± 5 % en poids et les trois microorganismes du mélange (b) étant chacun contenus en quantités de 33 ± 5 % en poids.

2. Cultures de départ selon la revendication 1, **caractérisées en ce qu'**elles contiennent :
(i) 40 à 60 % en poids du mélange (a),
(ii) 60 à 40 % en poids du mélange (b) et
(iii) 0 à 10 % en poids du mélange (c),
à condition que la somme des données de quantités soit de 100 % en poids.

3. Cultures de départ selon la revendication 1, **caractérisées en ce qu'**elles contiennent :
(i) 45 à 55 % en poids du mélange (a) et
(ii) 55 à 45 % en poids du mélange (b),
à condition que la somme des données de quantités soit de 100 % en poids.

4. Cultures de départ selon au moins l'une quelconque des revendications 1 à 3, **caractérisées en ce qu'**elles contiennent :
(i) 20 à 30 % en poids de *Streptococcus thermophilus,*
(ii) 5 à 15 % en poids de *Leuconostoc species,*
(iii) 5 à 10 % en poids de *Lactococcus lactis subsp. lactis biovar diacetylactis,*
(iv) 20 à 30 % en poids de *Lactococcus lactis subsp. lactis,*
(v) 20 à 30 % en poids de *Lactococcus lactis subsp. cremoris* et
(vi) 0 à 15 % en poids de *Bifido bacterium lactis B12,*
à condition que la somme des données de quantités soit de 100 % en poids.

5. Cultures de départ selon au moins l'une quelconque des revendications 1 à 3, **caractérisées en ce qu'**elles contiennent :
(i) 25 % en poids de *Streptococcus thermophilus,*
(ii) 12 % en poids de *Leuconostoc species,*
(iii) 13 % en poids de *Lactococcus lactis subsp. lactis biovar diacetylactis,*
(iv) 25 % en poids de *Lactococcus lactis subsp. lactis,*
(v) 25 % en poids de *Lactococcus lactis subsp. cremoris.*

6. Procédé de fabrication d'une masse de base de caillebotte présentant des propriétés gustatives améliorées, selon lequel
(a) du lait brut est soumis à un traitement thermique et la crème est séparée, de telle sorte qu'une masse de base de caillebotte non acidifiée se forme,
(b) le mélange ainsi obtenu est soumis à un traitement thermique jusqu'à ce qu'une dénaturation se produise,
(c) le produit dénaturé est mélangé avec des cultures de départ selon la revendication 1 et de la présure, et éventuellement
(d) la masse de base de caillebotte obtenue après la fin de la fermentation est ajustée à une teneur en matière sèche et en protéines définie.

7. Procédé selon la revendication 6, **caractérisé en ce que** la masse de base de caillebotte est soumise à un traitement thermique à une température de 85 à 90 °C pendant une durée de 5 à 10 minutes, et dénaturée.

8. Procédé selon les revendications 6 et/ou 7, **caractérisé en ce que** la masse dénaturée ainsi obtenue est mélangée à une température de 18 à 35 °C avec les cultures de départ et la présure.

9. Procédé selon au moins l'une quelconque des revendications 6 à 8, **caractérisé en ce que** la masse de base de caillebotte résultante est ajustée à une teneur en matière sèche de 15 à 20 % en poids et à une teneur en protéines de 10 à 15 % en poids.

10. Utilisation de mélanges de microorganismes selon la revendication 1 en tant que cultures de départ pour la fabrication de caillebotte.
